# EUROPÄISCHE PATENTSCHRIFT

(11) **EP 1 308 134 B1**
(45) Veröffentlichungstag und Bekanntmachung des Hinweises auf die Patenterteilung: **03.01.2007**
(21) Anmeldenummer: 02024098.2
(22) Anmeldetag: 29.10.2002
(51) Int. Cl.: A61B 17/70, A61M 25/10

(54) **Vorrichtung zum Aufrichten und Stabilisieren der Wirbelsäule**
Device for straightening and stabilising the spine
Dispositif de redressement et stabilisation du rachis

(30) Priorität: 03.11.2001 DE 10154163
(43) Veröffentlichungstag der Anmeldung: 07.05.2003
(73) Patentinhaber: Advanced Medical Technologies AG, 66620 Nonnweiler-Braunshausen (DE); Fürderer, Sebastian, Dr., 50354 Hürth-Efferen (DE)
(72) Erfinder: Fürderer, Sebastian, 50354 Hürth-Efferen (DE)
(74) Vertreter: Kruspig, Volkmar

(56) Entgegenhaltungen:
- WO-A-01/76514
- WO-A-98/56301
- US-A- 5 599 301
- US-A- 6 127 597

## Beschreibung

Die Erfindung betrifft eine Vorrichtung zum Aufrichten und Stabilisieren der Wirbelsäule, insbesondere zur Stabilisierung gebrochener Wirbel, mit einem verfestigtes Füllmaterial aufweisenden, in einem unter Kompression frakturierten Wirbelkörper oder zwischen benachbarten Wirbelkörpern anzuordnenden Stützkörper.

Es sind durch Benutzung Vorrichtungen zur Aufrichtung und Stabilisierung gebrochener Wirbel bekannt, welche einen Katheter aufweisen, der durch einen in den Pedikel des gebrochenen Wirbels gebohrten Kanal in den Wirbelinnenraum einführbar ist. Eine durch den Katheter in diesen Innenraum vorgeschobene Druckleitung weist an ihrem Ende einen aufweitbaren Druckballon auf, durch welchen ein durch Kompression gestauchter und ggf. gebrochener Wirbel wieder ausgedehnt und in seine ursprüngliche Form gebracht werden kann. Der danach entspannte und zusammen mit der Druckleitung zurückgezogene Ballon hinterlässt einen Hohlraum, in welchen sich, durch den Katheter hindurch, ein Knochenfüllermaterial einbringen lässt.

Aus der US-A-6127597 und der WO-A-0176514 sind Vorrichtungen der eingangs genannten Art bekannt, deren Stützkörper verfestigtes Füllmaterial aufweist, welches in einen im Körper verbleibenden, unter Bildung eines Hohlraums aufweitbaren Druckballon gefüllt ist. Die D1 US-A-6127597 beschreibt femer zur Anordnung zwischen benachbarten Wirbelkörpern anzuordnende, durch Innendruck aufweitbare Stützimplantate aus Metall, deren Festigkeit im aufgeweiteten Zustand ausreicht, um ohne weitere Stützeinrichtungen eine dauerhafte Stabilisierung der Wirbelsäule zu sichern.

Der vorliegenden Erfindung liegt die Aufgabe zugrunde, eine neue Vorrichtung zum Stabilisieren der Wirbelsäule der eingangs erwähnten Art zu schaffen, durch die sich bei geringem operativen Aufwand ein höherer Stabilisierungsgrad schneller als durch die bekannten derartigen Vorrichtungen erzielen lässt.

Die diese Aufgabe lösende Vorrichtung nach der Erfindung ist dadurch gekennzeichnet, dass der Stützkörper ein in dem Wirbelkörper bzw. zwischen den Wirbelkörpern durch Innendruck plastisch unter Beibehaltung der erreichten Endform aufweitbares Stützimplantat wie in Anspruch 1 definiert zur vorübergehenden Stabilisierung der Wirbelsäule vor der Verfestigung des Füllmaterials aufweist, wobei das verfestigte Füllmaterial einen Innenraum des aufgeweiteten Stützimplantats ausfüllt.

Ein solches Stützimplantat, das zur Anordnung im Innern eines unter Kompression frakturierten Wirbelkörpers oder, z.B. nach einer Bandscheibenresektion, zur Anordnung zwischen benachbarten Wirbelkörpern vorgesehen ist, lässt sich aufgrund seiner geringen Abmessungen vor der Aufweitung leicht an den Implantationsort bringen. Nach der Aufweitung ist sofort eine vorläufige Stabilisierung gewährleistet, indem das Stützimplantat seine bei der plastischen Aufweitung erreichte Endform beibehält. Das zunächst in flüssiger Form vorliegende Füllmittel kann unter geringem Druck in den entstandenen Hohlraum eingebracht werden und dort aushärten. Aufgrund der Wirkung des Stützimplantats braucht eine vollständige Aushärtung des Füllmittels nicht abgewartet zu werden.

Während mechanische Werkzeuge für die Erzeugung des Innendrucks denkbar sind, ist in der bevorzugten Ausführungsform der Erfindung eine Einrichtung vorgesehen, welche den Innendruck mit Hilfe eines Druckfluids erzeugt.

Vorzugsweise ist ein durch das Druckfluid beaufschlagbarer, im Innern des Stützimplantats angeordneter Druckballon vorgesehen.

Das aufweitbare Stützimplantat weist eine durchbrochene, in der Art von Streckmetall dehnbare Wand auf. Die Wand kann in der Art eines Balgs in Falten gelegt sein

Ein solches Stützimplantat lässt sich mit verhältnismäßig geringem Innendruck aufweiten, wobei durch die Schwächungen bzw. Falten die Stabilität des aufgeweiteten Implantats zwar gemindert ist, das Implantat aber trotzdem eine ausreichende Stützfunktion ausüben kann.

Insbesondere kann die Wand des aufweitbaren Stützimplantats Schwachstellen oder/und Falten in einer Anordnung derart aufweisen, dass sich das Stützimplantat zu einer gewünschten Form aufweitet. Zum Beispiel ist bei Anordnung eines solchen Stützimplantats zwischen benachbarten Wirbeln eine an einen Quader angenäherte Form wünschenswert.

In der bevorzugten Ausführungsform der Erfindung ist das aufweitbare Stützimplantat länglich ausgebildet, so dass es zum Anordnen am Implantationsort mit Hilfe eines Katheters bzw. einer Führungshülse geeignet ist. Insbesondere kann das aufweitbare Stützimplantat, und ggf. der Druckballon strumpfartig auf einer durch die Führungshülse hindurch zuführbaren Druckleitung aufsitzen, wobei der Druckballon zwischen dem Stützimplantat und der Druckleitung angeordnet ist und im nicht aufgeweiteten Zustand eine die Druckleitung umgebende Schlauchhülle bildet, die an ihren Enden um den Umfang der Druckleitung herum mit der Druckleitung druckdicht verbunden ist.

Vorzugsweise ist das Druckfluid inkompressibel, und es ist eine Einrichtung zur Messung der zugeführten Druckfluidmenge vorgesehen. Hierdurch besteht die Möglichkeit, über die zugeführte Menge den Aufweitungsgrad zu kontrollieren.

In weiterer vorteilhafter Ausgestaltung der Erfindung ist eine Änderungen des Fluiddrucks pro Zeit und der zugeführten Fluidmenge pro Zeit erfassende Überwachungseinrichtung zum Abbrechen der Druckbeaufschlagung bei Überschreiten vorgegebener Relationswerte der erfassten Größen vorgesehen. Eine solche Überwachungseinrichtung verhindert, dass es im Fall einer Zerstörung des Stützimplantats, z.B. infolge eines Materialfehlers, zu einem Austritt von unter hohem Druck stehendem Fluid in den Körper kommen kann.

Die Erfindung soll nun anhand von Ausführungsbeispielen und der beiliegenden, sich auf diese Ausführungsbeispiele beziehenden Zeichnungen näher erläutert werden. Es zeigen:
- Fig. 1: ein Ausführungsbeispiel für eine Vorrichtung nach der Erfindung mit einem auf einer Druckleitung aufsitzenden Stützimplantat,
- Fig. 2: ein Detail des Stützimplantats von Fig. 1,
- Fig. 3: einen Teil der Vorrichtung von Fig. 1, welcher in einen gebrochenen, gestauchten Wirbelkörper eingeführt ist,
- Fig. 4: den mit Hilfe der Vorrichtung von Fig. 1 wieder ausgedehnten Wirbelkörper von Fig. 3, und
- Fig. 5: ein weiteres Ausführungsbeispiel für ein in einer Vorrichtung nach der Erfindung verwendbares Stützimplantat.

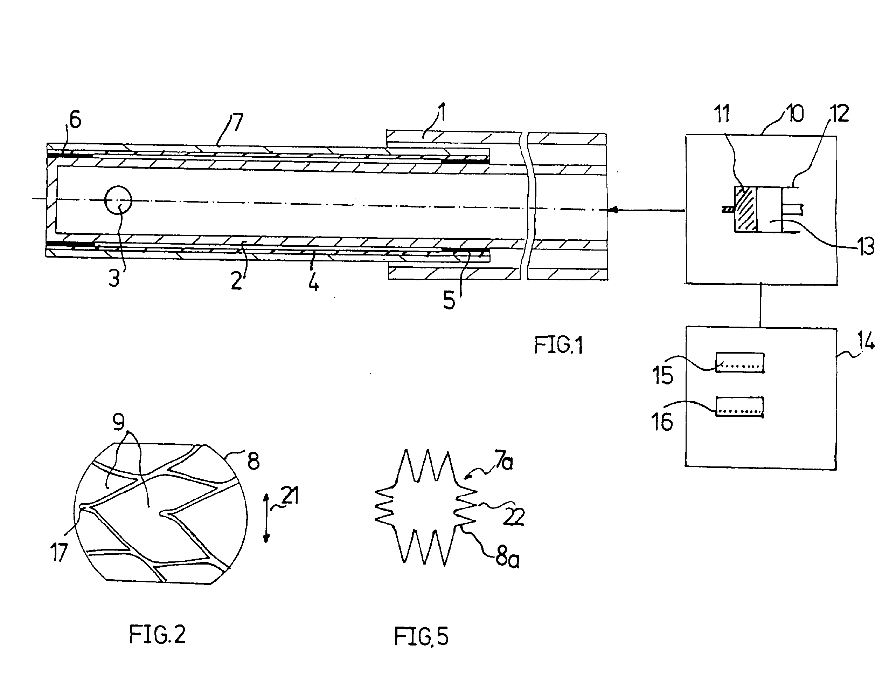

Mit dem Bezugszeichen 1 ist in der Fig. 1 eine Führungshülse bezeichnet, durch welche hindurch eine Druckleitung 2 mit einer Öffnung 3 für den Austritt eines Druckfluids geführt ist.

Auf der rundzylindrischen Druckleitung 2 sitzt bündig mit deren Ende strumpfartig eine elastische Schlauchhülle 4 auf, die an ihren Enden um den Umfang der Druckleitung 2 herum bei 5 und 6 druckdicht mit der Druckleitung verklebt ist. Statt einer solchen Verklebung wäre es denkbar, die elastische Schlauchhülle 4 an den Enden mit Hilfe von Ringen an die Druckleitung anzupressen.

Auf der elastischen Schlauchhülle 4 sitzt strumpfartig ein hohlzylindrisches Stützimplantat 7 auf, dessen die Zylindermantelfläche bildende Wand 8, wie aus Fig. 2 hervorgeht, netzartig mit Druckbrüchen 9 ausgebildet ist, wobei Netzadern bei 17 spitzwinklig zusammenlaufen. Die Wand 8 lässt sich tangential gemäß Pfeil 21 in der Art von Streckmetall ausdehnen und das Stützimplantat 7 dadurch radial aufweiten.

An ihrem der Hülle 4 bzw. dem Stützimplantat 7 entgegengesetzten Ende steht die Druckleitung 2 in Verbindung mit einer schematisch dargestellten Einrichtung 10 für die Zuführung eines inkompressiblen Druckfluids 11, wobei diese Einrichtung einen Druckzylinder 12 und einen Kolben 13 umfasst. Der Kolben 13 kann von Hand, vorzugsweise mit Hilfe eines Schraubmanometers, oder einen Motorantrieb bewegbar sein.

Mit dem Bezugszeichen 14 ist eine schematisch dargestellte Steuer- und Überwachungseinrichtung bezeichnet, welche eine Druckanzeige 16 und eine Anzeige 17 für die zugeführte Druckfluidmenge aufweist.

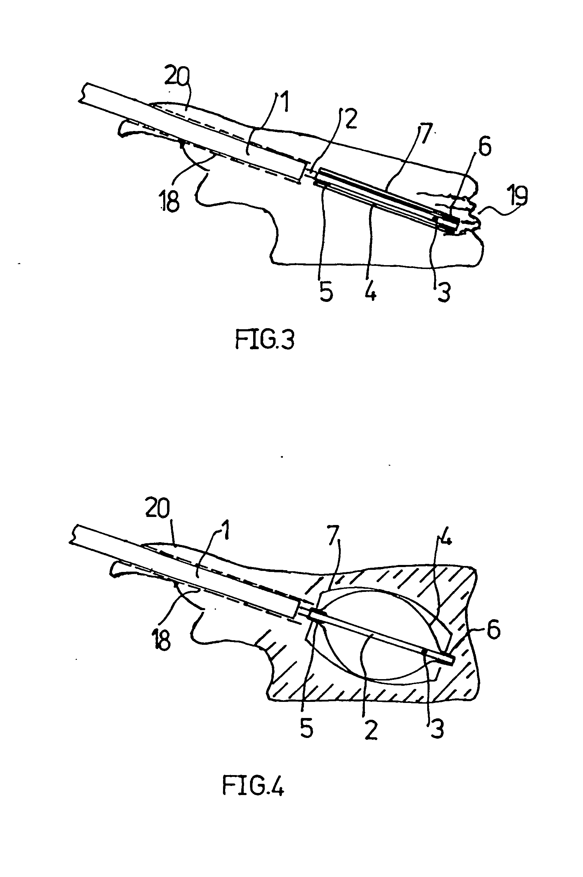

Anhand der Fig. 3 und 4 soll nun die Funktionsweise der in den Fig. 1 und 2 beschriebenen Vorrichtungen erläutert werden.

Zwecks Stabilisierung eines gebrochenen Wirbels wird zunächst durch den Pedikel 20 hindurch ein Kanal 18 gebohrt, wobei sich hierzu ein Katheter und ein durch den Katheter geführtes Bohrwerkzeug verwenden lässt. In den Kanal 18 wird, wie in den Fig. 3 und 4 gezeigt, die Führungshülse 1 eingesetzt, durch welche hindurch die Druckleitung 2 mit dem Stützimplantat 7 voran in den Innenraum des gestauchten, bei 19 Stauchfalten aufweisenden Wirbels vorgeschoben werden kann.

Mit Hilfe der Einrichtung 10 wird das inkompressible Druckfluid 11 in die Druckleitung 2 eingedrückt, wobei das Druckfluid 11 aus der Öffnung 3 austritt und die elastische Schlauchhülle 4 zu einem Ballon ausdehnt. Die sich ausdehnende Hülle bzw. der Ballon 4 weitet das Stützimplantat 7, wie in Fig. 4 gezeigt ist, auf, wobei sich die Wand 8 des Stützimplantats plastisch in Richtung des in Fig. 2 gezeigten Pfeils 21 unter Aufweitung der spitzen Winkel zwischen den Netzadern bei 17 tangential ausdehnt.

Während der Aufweitung lässt sich die zugeführte Druckfluidmenge an der Anzeige 17 der Steuer- und Überwachungseinrichtung 14 ablesen und somit das Ausmaß der erreichten Aufweitung bestimmen. Die Aufweitung bzw. Druckfluidzuführung wird abgeschlossen, wenn ein vorbestimmter Grenzwert der zugeführten Druckfluidmenge erreicht ist.

Die Steuer- und Überwachungseinrichtung 14 sichert ferner, dass die Druckbeaufschlagung sofort abgebrochen wird, falls es während der Aufweitung, z.B. durch einen Materialfehler, zum Reißen des Ballons 4 und zum Austritt von Druckfluid aus dem Wirbel kommen sollte, was sich dadurch bemerkbar macht, dass die zugeführte Druckfluidmenge bei gleichbleibendem oder nur schwach ansteigendem Druck zeitlich stark anwächst.

Nach Erreichen der erforderlichen Aufweitung erfolgt eine Absaugung des Druckfluids über die im nahe dem tiefsten Punkt des Ballons 4 gelegene Öffnung 3. Die Druckleitung 2 mit dem entleerten Druckballon bzw. der entleerten Schlauchhülle 4 lässt sich nun durch die Führungshülse 1 hindurch zurückziehen.

Das plastisch verformte Stützimplantat 7 behält seine Form bei und stützt den Wirbel ab, so dass er die in Fig. 4 gezeigte Form behält und die bei 19 gezeigten Beschädigungen ausheilen können. In den Innenraum des Stützimplantats wird ein Füllmittel eingegeben.

Es wird nun auf Fig. 5 Bezug genommen, wo im Querschnitt ein weiteres Auführungsbeispiel für ein Stützimplantat 7a gemäß der Erfindung gezeigt ist. Das Stützimplantat 7a weist in seiner Wand 8a Falten 22 auf, wobei die Falten auf gegenüberliegenden Seiten unterschiedlich lang sind, so dass sich für das aufgeweitete Implantat im Querschnitt eine Rechteckform ergibt.

Als Druckfluid wird in dem oben beschriebenen Ausführungsbeispiel eine Röntgenkontrastmittel enthaltende Kochsalzlösung verwendet.

Es versteht sich, das in einem gebrochenen Wirbel zwei der beschriebenen Stützimplantate eingeführt werden können und im Regelfall einzuführen sind, wobei jeder der beiden Pedikel durchbohrt wird, um einen Katheterkanal zu bilden.

## Patentansprüche

1. Vorrichtung zum Aufrichten und Stabilisieren der Wirbelsäule, insbesondere zur Stabilisierung gebrochener Wirbel, mit einem verfestigbarem Füllmaterial und einem in einen, unter Kompression frakturierten Wirbelkörper oder zwischen benachbarten Wirbelkörpern anzuordnenden Stützkörper,
**dadurch gekennzeichnet, daß**
der Stützkörper ein in dem Wirbelkörper oder zwischen den Wirbelkörpern durch Innendruck plastisch unter Beibehaltung der erreichten Endform aufweitbares Stützimplantat (7,7a) zur vorübergehenden Stabilisierung der Wirbelsäule vor der Verfestigung des Füllmaterials umfaßt, wobei das verfestigte Füllmaterial einen Innenraum des aufgeweiteten Stützimplantats ausfüllt und das aufweitbare Stützimplantat (7,7a) eine durchbrochene, in der Art von Streckmetall dehnbare Wand (8,8a) aufweist.

2. Vorrichtung nach Anspruch 1,
**dadurch gekennzeichnet, daß**
Einrichtungen (10) für die Erzeugung des Innendrucks mit Hilfe eines Druckfluids (11) vorgesehen sind.

3. Vorrichtung nach Anspruch 2,
**dadurch gekennzeichnet, daß**
ein durch das Druckfluid (11) beaufschlagbarer, im Innern des Stützimplantats anzuordnender Druckballon (4) vorgesehen ist.

4. Vorrichtung nach einem der Ansprüche 1 bis 3,
**dadurch gekennzeichnet, daß**
das aufweitbare Stützimplantat (7) eine längliche, zur Anordnung am Implantatsort mit Hilfe einer Führungshülse (1) geeignete Form aufweist.

5. Vorrichtung nach Anspruch 3,
**dadurch gekennzeichnet, daß**
das aufweitbare Stützimplantat, und gegebenenfalls der Druckballon (4), strumpfartig auf einer durch die Führungshülse (1) hindurch zuführbaren Druckleitung (2) aufsitzen.

6. Vorrichtung nach einem der Ansprüche 1 bis 5,
**dadurch gekennzeichnet, daß**
das Druckfluid inkompressibel und eine Einrichtung (14) zur Messung der zugeführten Druckfluidmenge vorgesehen ist.

7. Vorrichtung nach einem der Ansprüche 1 bis 6,
**dadurch gekennzeichnet, daß**
eine die Änderung des Fluiddrucks pro Zeit und die zugeführte Druckfluidmenge pro Zeit erfassende Steuer- und Überwachungseinrichtung (14) vorgesehen ist, welche die Druckbeaufschlagung abbricht, wenn das Verhältnis der zugeführten Druckfluidmenge pro Zeit zu der Änderung des Fluiddrucks pro Zeit einen vorbestimmten Wert überschreitet.

## Claims

1. A device for straightening and stabilizing the spine, in particular for stabilizing broken vertebrae, having a filler material capable of solidifying and a supporting body to be arranged in a vertebral body fractured under compression or between adjacent vertebral bodies,
**characterized in that**
said supporting body comprises supporting implant (7, 7a) plastically expandable in the vertebral body or between vertebral bodies by means of internal pressure while maintaining the acquired end shape for preliminarily stabilizing the spine before the solidification of the filler material, the solidified filler material filling an inner space of the expanded supporting implant, and the expandable supporting implant (7, 7a) having a perforated wall (8, 8a) expandable in the form of an expanded metal.

2. The device of claim 1,
**characterized in that**
means (10) are provided for generating the internal pressure by means of a pressure fluid (11).

3. The device of claim 2,
**characterized in that**
a pressure balloon (4) to be arranged inside the supporting implant is provided which can be pressurized by said pressure fluid (11).

4. The device according to any one of claims 1 through 3,
**characterized in that**
said expandable supporting implant (7) has an oblong shape suitable for being positioned at the implant site by means of a guide sheath (1).

5. The device of claim 3,
**characterized in that**
said expandable supporting implant and, as the case may be, the pressure balloon (4), rests in the manner of a stocking on a pressure line (2) which can be introduced through said guide sheath (1).

6. The device of any one of claims 1 through 5,
**characterized in that**
said pressure fluid is incompressible and a means (14) is provided for measuring the supplied amount of pressure fluid.

7. The device of any one of claims 1 through 6,
**characterized in that**
a control and monitoring device (14) detecting the change in the fluid pressure over time and the amount of pressure fluid supplied over time is provided, which interrupts the pressurization when the ratio of the supplied pressure fluid amount over time and the change in the fluid pressure over time exceeds a predetermined value.

## Revendications

1. Dispositif de redressement et de stabilisation de la colonne vertébrale, en particulier pour stabiliser des vertèbres fracturées, comportant un matériau de remplissage susceptible de se solidifier et un corps de soutien à agencer dans un corps de vertèbre fracturé sous compression ou entre des corps de vertèbres voisins, **caractérisé en ce que** le corps de soutien comprend un implant de soutien (7, 7a) susceptible d'être évasé de manière plastique dans le corps de vertèbre ou entre les corps de vertèbres par pression intérieure en conservant la forme finale atteinte pour stabiliser provisoirement la colonne vertébrale avant la solidification du matériau de remplissage, le matériau de remplissage solidifié remplissant un espace intérieur de l'implant de soutien évasé et l'implant de soutien (7, 7a) susceptible d'être évasé présentant une paroi (8, 8a) ajourée extensible à la manière de métal déployé.

2. Dispositif selon la revendication 1, **caractérisé en ce qu'**il est prévu des systèmes (10) pour produire la pression intérieure à l'aide d'un fluide de pression (11).

3. Dispositif selon la revendication 2, **caractérisé en ce qu'**il est prévu un ballon de pression (4) sollicité par le fluide de pression (11) et à agencer à l'intérieur de l'implant de soutien.

4. Dispositif selon l'une des revendications 1 à 3, **caractérisé en ce que** l'implant de soutien (7) susceptible d'être évasé présente une forme allongée qui se prête à l'agencement au lieu d'implantation à l'aide d'une douille de guidage (1).

5. Dispositif selon la revendication 3, **caractérisé en ce que** l'implant de soutien susceptible d'être évasé et, le cas échéant, le ballon de pression (4) sont placés à la manière d'une chaussette sur une conduite de pression (2) que l'on peut faire passer à travers la douille de guidage (1).

6. Dispositif selon l'une des revendications 1 à 5, **caractérisé en ce que** le fluide de pression est incompressible et **en ce qu'**il est prévu un système (14) pour mesurer la quantité de fluide de pression amenée.

7. Dispositif selon l'une des revendications 1 à 6, **caractérisé en ce qu'**il est prévu un système de commande et de surveillance (14) détectant la variation de la pression du fluide par unité de temps et la quantité de fluide de pression amenée par unité de temps, système qui interrompt l'alimentation en pression lorsque le rapport entre la quantité de fluide de pression amenée par unité de temps dépasse une valeur prédéterminée de pression de fluide par unité de temps.
